# EUROPEAN PATENT SPECIFICATION

(11) **EP 2 055 770 B1**
(45) Date of publication and mention of the grant of the patent: **10.08.2011**
(21) Application number: 07119544.0
(22) Date of filing: 29.10.2007
(51) Int. Cl.: C12M 1/42, C12N 1/12

(54) **Apparatus and method for growing algae by ionizing radiation**
Vorrichtung und Verfahren zur Algenzüchtung mittels ionisierender Strahlung
Appareil et procédé pour faire pousser des algues par radiations ionisantes

(43) Date of publication of application: 06.05.2009
(73) Proprietor: Atomic Energy Council - Institute of Nuclear Energy Research, Lungtan, Taoyuan, Taiwan (TW)
(72) Inventor: Chen, Yean-Chang, Keelung City 203 (TW); Chen, Kuan-Yin, Taipei County 242 (TW); Chen, Chia-Chieh, Taoyuan County 325 (TW); Lin, Bin, Hualien County 970 (TW); Liu, Hsueh-Hsuan, Kaohsiung City 804 (TW); Lee, Meng-Chou, Taipei City 116 (TW); Lin, Wuu-Jyh, Taoyuan County 325 (TW); Fu, Ying-Kai c/o Atomic Energy Council, No. 1000, Wenhua Rd., Longtan Shiang Taoyuan County 325 (TW); Shen, Lie-Hang, Zhongli City Taoyuan County 320 (TW); Hwang, Wen-Song, Taoyuan County 325 (TW); Kuo, Ming-Chao, Hsinchu City 300 (TW); Chen, Wei-His, Taipei City 115 Taiwan (CN); Wu, Te-Hsing, Taoyuan County 330 Taiwan (CN)
(74) Representative: Beck & Rössig European Patent Attorneys

(56) References cited:
- EP-A- 0 999 265
- JP-A- 2001 231 387
- JP-A- 2006 230 211
- US-A- 4 324 067

## Description

### BACKGROUND OF INVENTION

### 1. FIELD OF INVENTION

The present invention relates to an apparatus and method for growing algae by ionizing radiation.

### 2. RELATED PRIOR ART

Algae can be used to make medicine and food, process wastewater and provide energy. Therefore, there is a considerable need for algae.

An apparatus for and a method of growing algae by ionizing radiation is known from JP 2001231387 A. More specifically, JP 2001231387 A discloses a culturing tool having a seed thread for attaching spores of algae and growing the spores and a frame body to which the seed thread is attached. The frame body has an upper frame part and a lower frame part arranged at a prescribed distance onto which the seed thread is wound. This prior art tool for culturing algae is said to be capable of heightening the efficiency of initial growing and intermediate growing, and facilitating the maintenance after making a seaweed bed, and further to enable a method for efficiently growing algae.

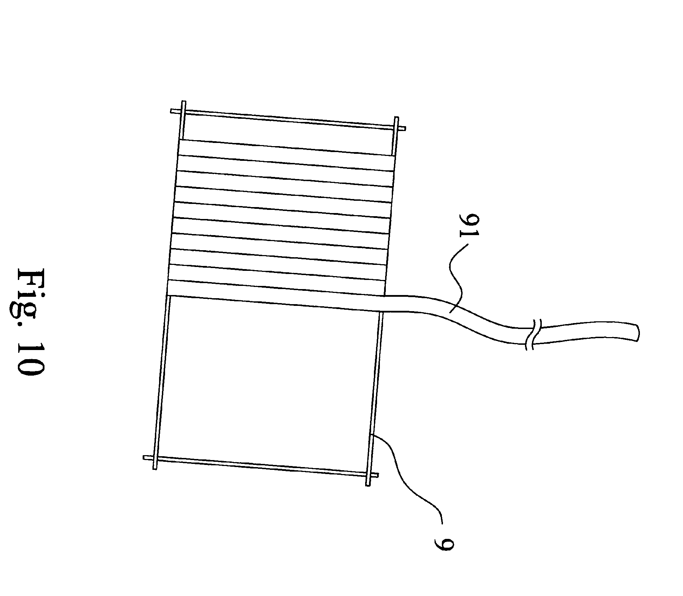

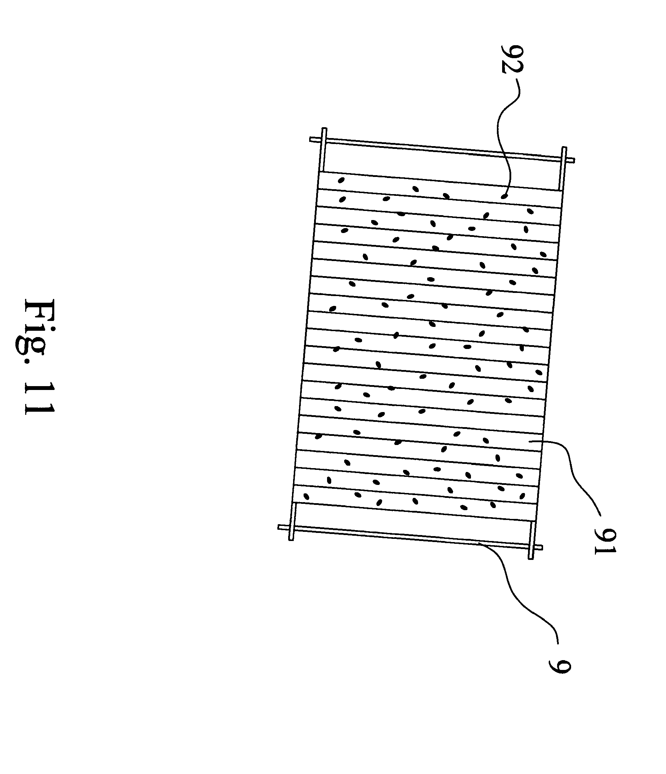

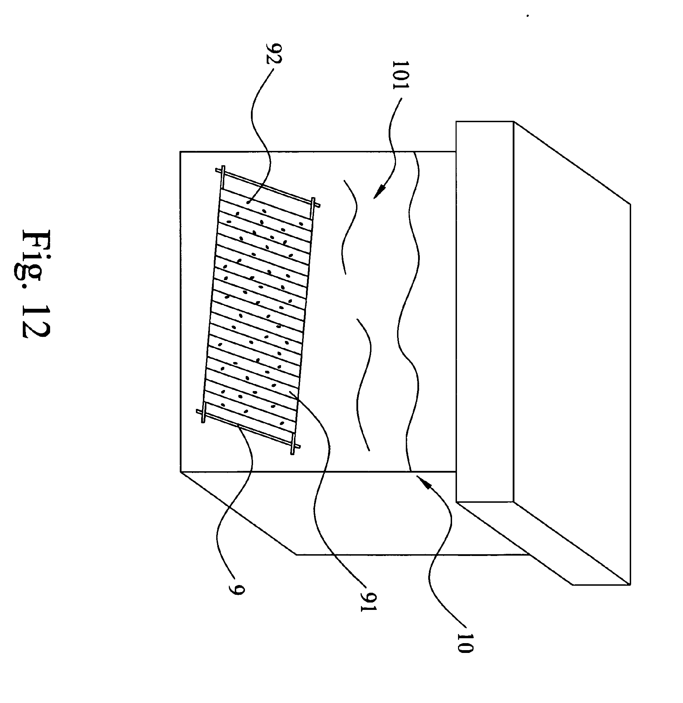

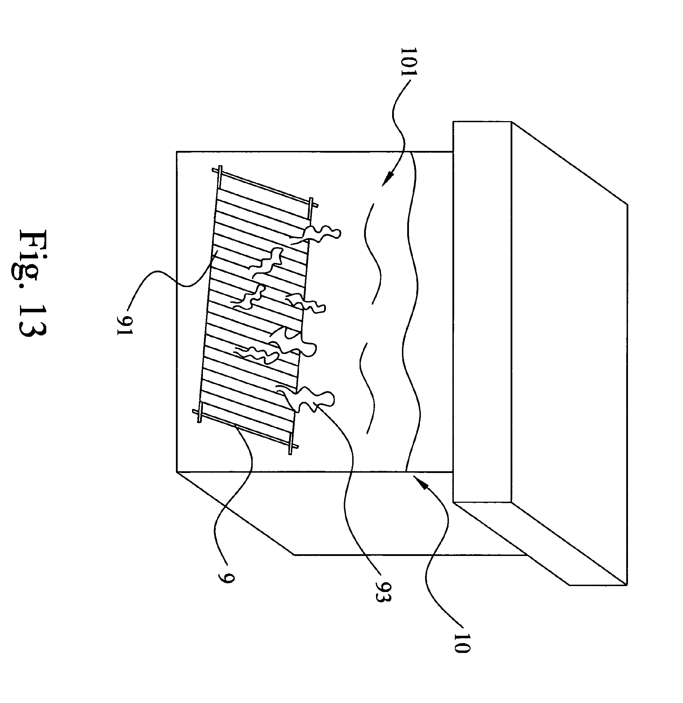

Referring to Figs. 10 through 13, a conventional apparatus for growing algae includes a flat rack 9, a cotton rope 91 wound on the flat rack 9, and a culture plate 10 for containing seawater 101. Spores 92 of algae are spread on segments of the cotton rope 91 located on an upper side of the flat rack 9. Then, the spores 92 planed in the cotton rope 91 are submerged in the seawater 101. The spores 92 will grow to be bodies 93 referring to Fig. 13. However, the throughput of the production of the algae is low for two reasons. Firstly, the growth of the algae is low. Secondly, a small amount of spores 92 planted. The spores 92 are only spread on the segments of the cotton rope 91 located on the upper side of the flat rack 9 because segments of the cotton rope 91 are laid on an upper side of the culture plate 10 and cannot be irradiated by the sunlight.

The present invention is therefore intended to obviate or at least alleviate the problems encountered in prior art.

### SUMMARY OF INVENTION

The primary objective of the present invention is to provide an apparatus for growing algae quickly.

To achieve the foregoing objective the present invention provides an apparatus according to claim 1 and a method according to claim 7.

Advantageous embodiments are laid down in further claims.

Further objectives, advantages and features of the present invention will become apparent from the following description referring to the attached drawings.

### BRIEF DESCRIPTION OF DRAWINGS

The present invention will be described via detailed illustration of the preferred embodiment referring to the drawings.
Fig. 1 is a perspective view of an apparatus for growing algae by ionizing radiation according to the preferred embodiment of the present invention.
Fig. 2 is an exploded view of the apparatus shown in Fig. 1.
Fig. 3 is a perspective view of an adherent unit wound on a 3-dimensional rack used in the apparatus shown in Fig. 1.
Fig. 4 shows sea water contained in the apparatus shown in Fig. 1.
Fig. 5 is a partial cross-sectional view of the apparatus shown in Fig. 4.
Fig. 6 is a cross-sectional view of the apparatus shown in Fig. 4.
Fig. 7 is a perspective view of an outdoor running-water pool for the growth of algae cultured by the apparatus shown in Fig. 5.
Fig. 8 is a cross-sectional view of the outdoor running-water pool shown in Fig. 7.
Fig. 9 is a bar chart for showing different growth rates of algae cultured under different degrees of ionizing radiation.
Fig. 10 is a top view of a cotton rope wound on a flat rack used in a conventional apparatus for growing algae.
Fig. 11 is a top view of spores of algae spread on the cotton rope shown in Fig. 10.
Fig. 12 shows a culture plate for containing seawater for submerging the spores shown in Fig. 11.
Fig. 13 shows algae grown from the spores shown in Fig. 12.

### DETAILED DESCRIPTION OF PREFERRED EMBODIMENT

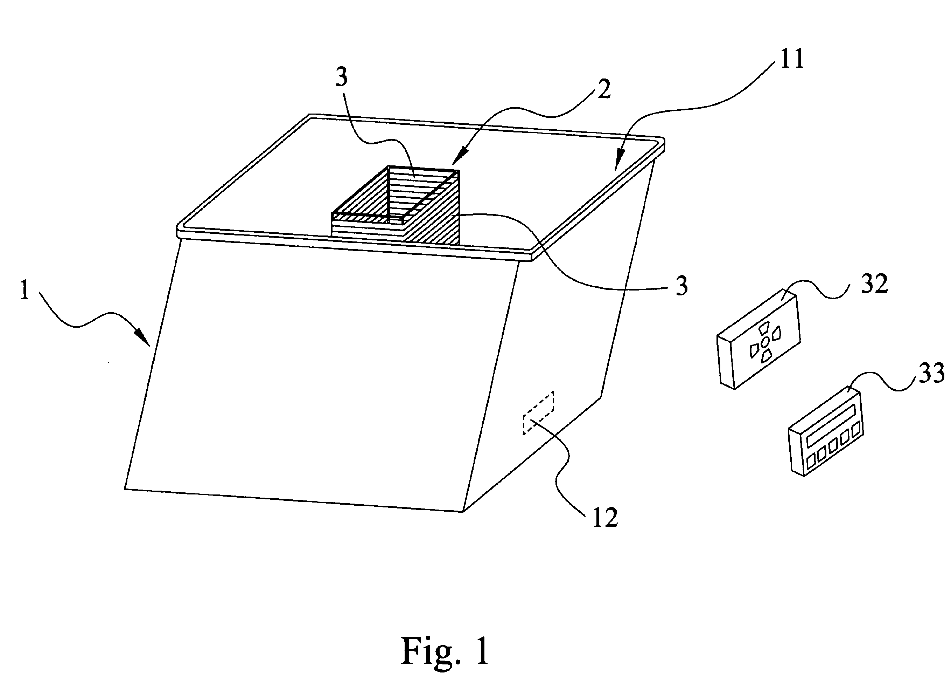

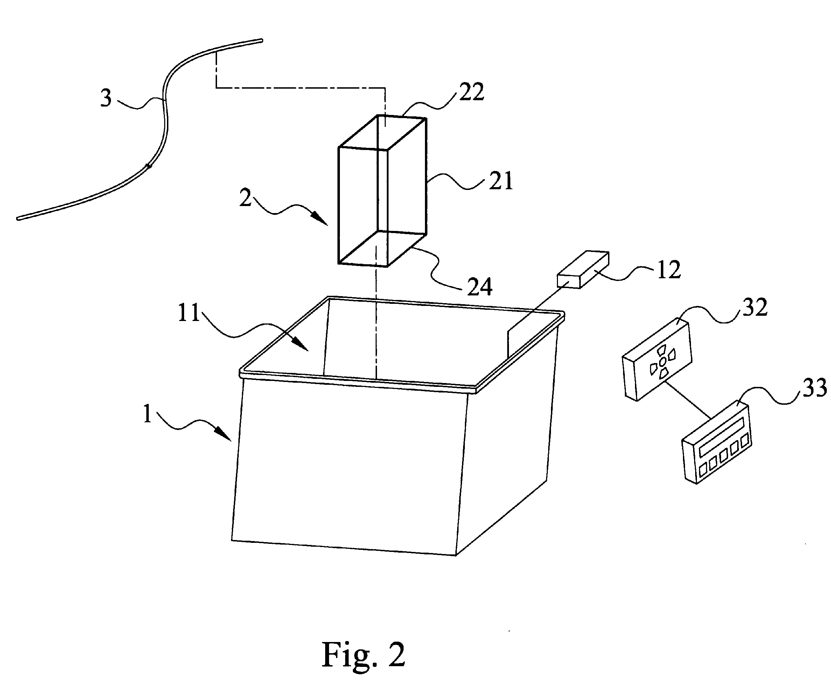

Referring to Figs. 1 and 2, an apparatus for growing algae by ionizing radiation includes a container 1, a 3-dimensional rack 2 ("3-D rack"), an adherent element 3, an ionizing radiation element 32 and a timepiece 33 according to the preferred embodiment of the present invention.

The container 1 includes a space 11 defined therein. A filter 12 is disposed in the space 11. The container 1 is preferably made of polyvinylchloride. The volume of the container 1 is about 120 liters.

The 3-D rack 2 is preferably made of stainless steel. The 3-D rack 2 is a square rack including four bars 21, four bars 22 and four bars 24. The length of the bars 21 is 40 cm. The length of the bars 22 is 30 cm. The length of the bars 24 is 30 cm. The diameter of the bars 21, 22 and 24 is about 0.7 cm.

The adherent element 3 is preferably a cotton rope. The diameter of the adherent element 3 is about 1 cm. The length of the adherent element 3 is about 33 m.

The ionizing radiation element 32 preferably provides Co-60 γ radiation.

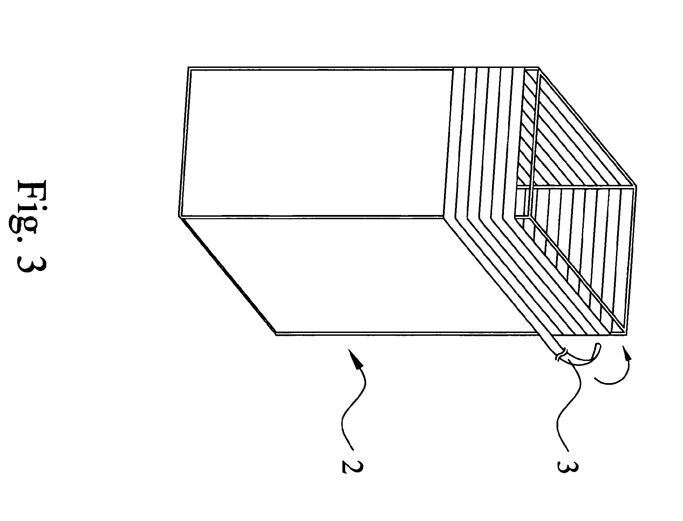

Referring to Fig. 3, the adherent element 3 is wound on the 3-D rack 2. More particularly, the adherent element 3 is wound on the bars 21.

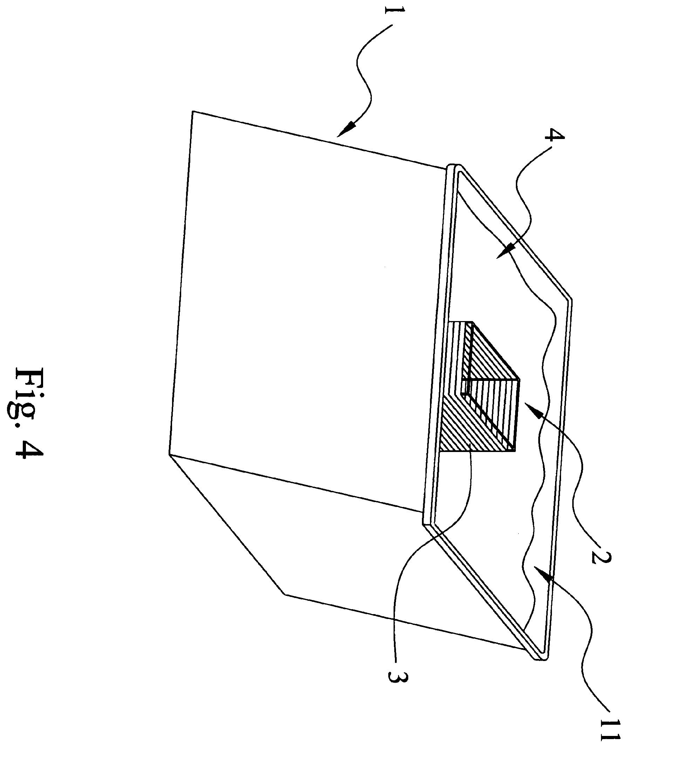

Referring to Fig. 4, about 100 liters of medium 4 are filled in the container 1. The medium 4 is preferably seawater. The adherent element 3 wound on the 3-D rack 2 is completely submerged in the medium 4. As the adherent element 3 is wound on the bars 21, the entire length thereof can be irradiated by sunlight when it is submerged in the medium 4.

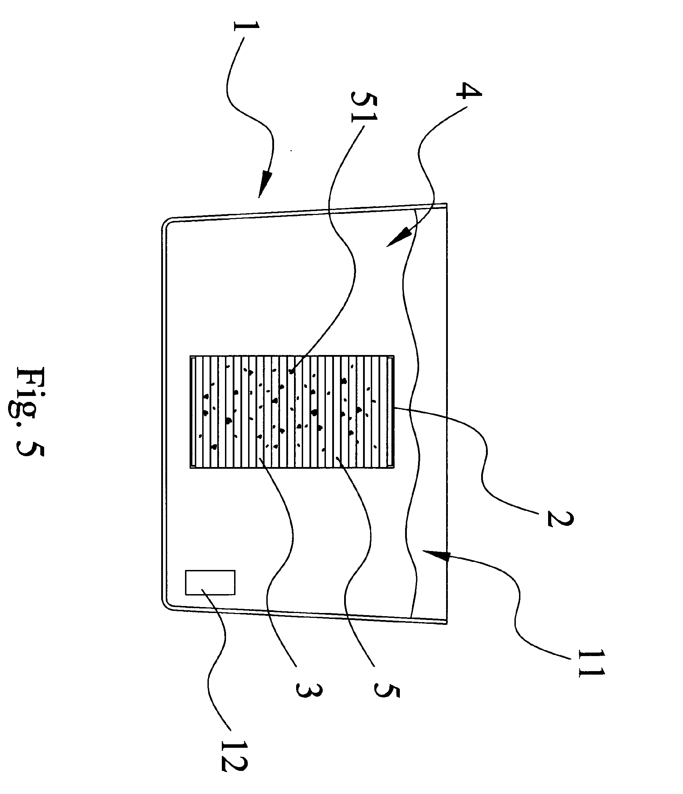

Referring to Fig. 5, a large number of spores 5 of algae are implanted in the entire length of the adherent element 3. As mentioned above, the filter 12 is disposed in the container 1. The container 1 is disposed in a green house where the spores 5 can be irradiated by sunlight. After 1 day or 2, the cells of the spores 5 are completely adhered to the adherent element 3. On the 15^{th} day after the planting of the spores 5, some of the spores 5 grow to be sprouts 51 about 0.5 to 1.0 mm long. The filter 12 is activated to filter the seawater 4 and generate a current. The flow rate of the current is about 50 to 75 ml/sec. That is, the algae are subjected to running-water culturing.

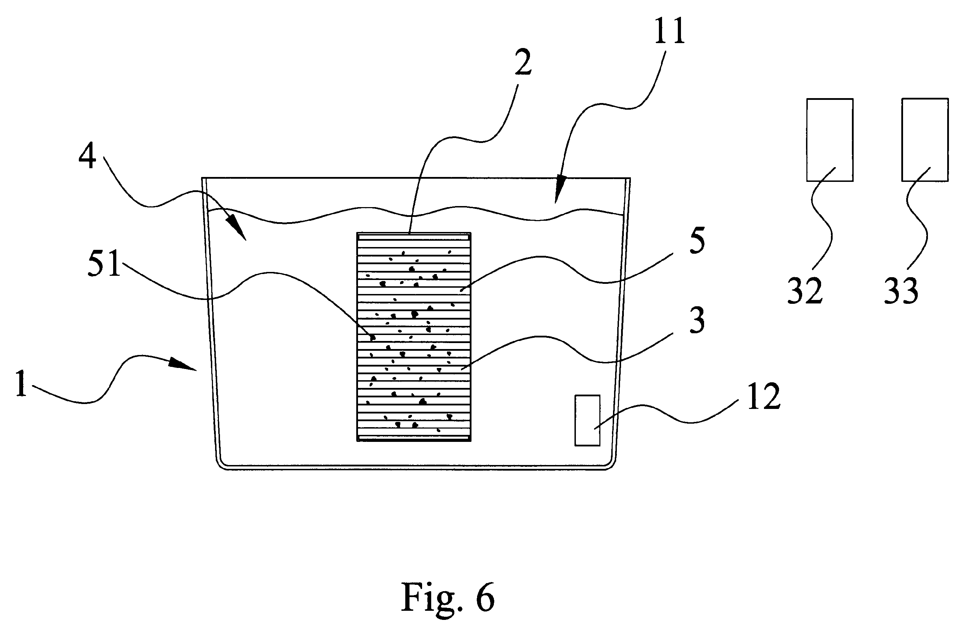

Referring to Fig. 6, the ionizing radiation element 32 is activated to irradiate the spores 5 and the sprouts 51. The control over the radiation dose is done via the control over the radiation time. The control over the radiation time is conducted with the timepiece 33. If the algae are ulvaceae, the optimal dose of the ionizing radiation is 15 Gy every three weeks. The 15 Gy is provided in two rounds under the control of the timepiece 33. If the ionizing radiation element 3 provides the ionizing radiation at a power of 5 Gy/min, the total time for the ionizing radiation is totally 3 min for every three weeks. The 3 min are divided into two periods of time.

Alternatively, the spores 5 and the sprouts 51 can be irradiated in a conventional process. In the conventional process, an ionizing radiation element like the ionizing radiation element 32 is disposed in a pool of water. The spores 5 and the sprouts 51 adhered to the adherent element 3 wound on the 3-D frame 2 are disposed in a watertight box. Then, the watertight box is submerged in the pool of water. Finally, the ionizing radiation element is activated to irradiate the spores 5 and the sprouts 51.

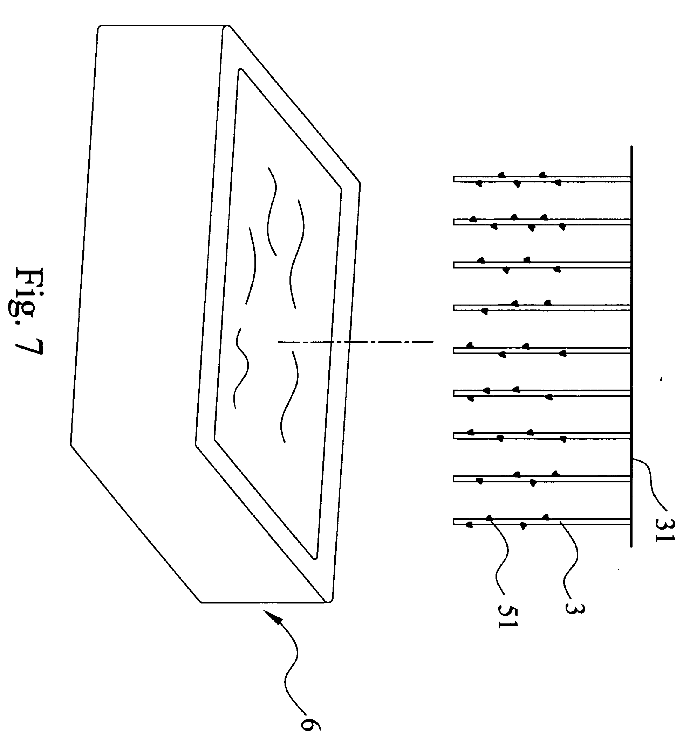

Referring to Fig. 7, on the 40^{th} day, most of the spores 5 have grown to be the sprouts 51. The length of the sprouts 51 is about 3.0 to 5.0 mm. The adherent element 3 is removed from the 3-D frame 2. The adherent element 3 is cut into segments of 1.0 m. The segments are hung in parallel on a rope 31. The diameter of the rope 31 is about 0.6 cm. Then, the segments are submerged in seawater filled in an outdoor running-water pool 6. The outdoor running-water pool 6 is about 4.4 long, 2.2 mm wide and 1.3 meter high. The seawater is about 0.9 m deep so that the volume of the seawater is about 8.7 m³. The flow rate of the seawater is about 300 ml/sec. Thus, running-water deutero-culturing of the spores 5 and sprouts 51 is conducted.

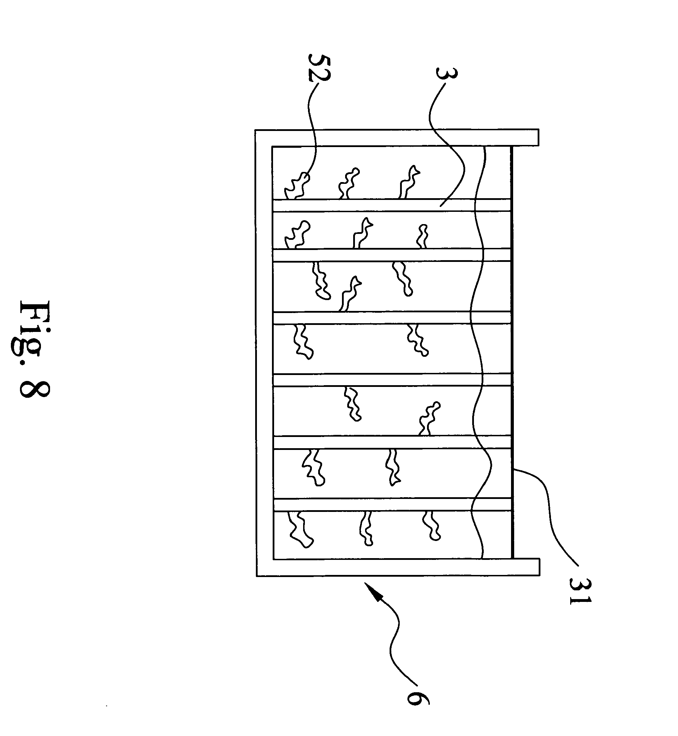

Referring to Fig. 8, almost all of the spores 5 have grown to be bodies 52 or algae of an appropriate length such as 10 mm. The bodies 52 are picked from the segments, washed with filtered seawater and baked in an oven at 70 degrees Celsius, thus removing the filtered seawater from the bodies 52. Finally, the dried bodies 52 are weighed to calculate the biomass thereof.

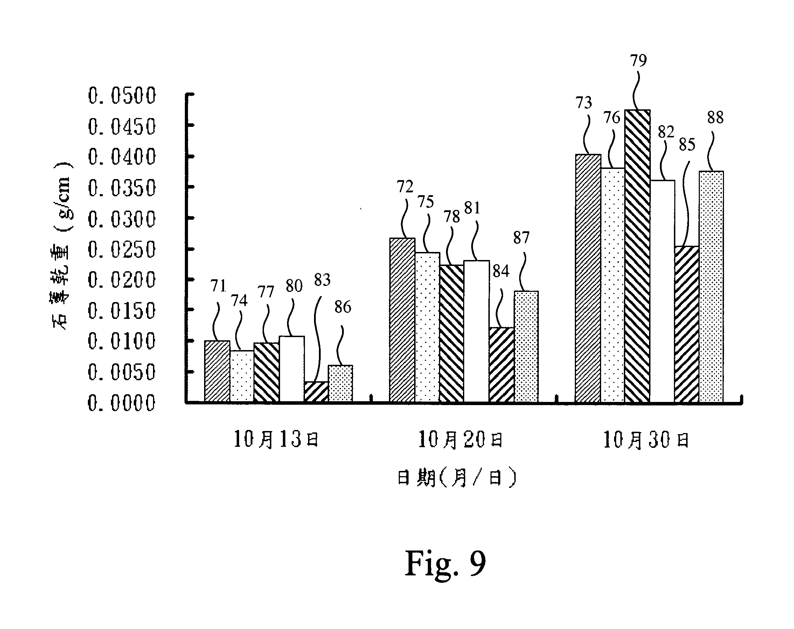

Referring to Fig. 9, an experiment was conducted from 24 August 2007 to 30 October 2007. Ulvaceae was grown in methods according to five embodiments of the present invention and in a conventional method. Results were measured. Spores 5 of the ulvaceae were planted in the adherent element 3 on 24 August 2007.

According to the first embodiment, the Co-60 γ radiation was conducted on 7 September 2007. A specimen 71 was picked on 13 October 2007. The biomass of the specimen 71 is 0.0101 g/cm. A specimen 72 was picked on 20 October 2007. The biomass of the specimen 72 is 0.0267 g/cm. A specimen 73 was picked on 30 October 2007. The biomass of the specimen 73 is 0.0404 g/cm.

According to the second embodiment, the Co-60 γ radiation was conducted on 7 September 2007 and 4 October 2007. A specimen 74 was picked on 13 October 2007. The biomass of the specimen 74 is 0.0085 g/cm. A specimen 75 was picked on 20 October 2007. The biomass of the specimen 75 is 0.0244 g/cm. A specimen 75 was picked on 30 October 2007. The biomass of the specimen 75 is 0.0381 g/cm.

According to the third embodiment, the Co-60 γ radiation was conducted on 7 September 2007 and 28 September 2007. A specimen 77 was picked on 13 October 2007. The biomass of the specimen 77 is 0.0096 g/cm. A specimen 78 was picked on 20 October 2007. The biomass of the specimen 78 is 0.0223 g/cm. A specimen 79 was picked on 30 October 2007. The biomass of the specimen 79 is 0.0475 g/cm.

According to the fourth embodiment, the Co-60 γ radiation was conducted on 7 September 2007, 21 September and 4 October 2007. A specimen 80 was picked on 13 October 2007. The biomass of the specimen 80 is 0.0108 g/cm. A specimen 81 was picked on 20 October 2007. The biomass of the specimen 81 is 0.0231 g/cm. A specimen 82 was picked on 30 October 2007. The biomass of the specimen 82 is 0.0404 g/cm.

According to the fifth embodiment, the Co-60 γ radiation was conducted on 7 September 2007, 14 September 2007, 21 September 2007, 28 September 2007 and 4 October 2007. A specimen 83 was picked on 13 October 2007. The biomass of the specimen 83 is 0.0033 g/cm. A specimen 84 was picked on 20 October 2007. The biomass of the specimen 84 is 0.0121 g/cm. A specimen 85 was picked on 20 October 2007. The biomass of the specimen 85 is 0.0255 g/cm.

In the conventional method embodiment, the Co-60 γ radiation was never conducted. A specimen 86 was picked on 13 October 2007. The biomass of the specimen 86 is 0.0059 g/cm. A specimen 87 was picked on 20 October 2007. The biomass of the specimen 87 is 0.0182 g/cm. A specimen 88 was picked on 20 October 2007. The biomass of the specimen 88 is 0.0377 g/cm.

The biomasses of the specimens 73, 76 and 79 grown according to the first, second and third embodiments of the present invention are larger than that of the specimen 88 grown in the conventional method. The biomasses of the specimens 73 and 76 grown according to the first and second embodiments of the present invention are however close to that of the specimen 88 grown in the conventional method. The biomass of the specimen 79 grown according to the third embodiment of the present invention is the largest. The biomasses of the specimens 82 and 85 grown according to the fourth and fifth embodiments of the present invention are smaller than that of the specimen 88 grown in the conventional method.

It can be inferred that insufficient dosing of the Co-60 γ radiation is good for the growth of the ulvaceae to a limited extent. It can also be inferred that excessive dosing of the Co-60 γ radiation is bad for the growth of the ulvaceae.

## Claims

1. An algae-growing apparatus comprising:
a container (1) for containing medium for the growth of algae;
a filter (121) disposed in the container (1); said filter being adapted to be activated to filter said medium in said container (1) and generate a current of said medium;
a 3-dimensional rack (2) disposed in the container (1);
an adherent element (3) wound on the 3-dimension rack (2) so that spores of the algae can be planted in the adherent element (3);
an ionizing radiation element (32) disposed in the container (1) for irradiating the spores; and
a timepiece (33) connected to the ionizing radiation element (32).

2. The apparatus according to claim 1, wherein the medium is seawater.

3. The apparatus according to claim 1 or 2, wherein the 3-dimensional rack (3) comprises a plurality of beams and bars (21, 22, 24) connected to one another.

4. The apparatus according to one of claims 1 to 3, wherein the adherent element (3) is a cotton rope.

5. The apparatus according to one of claims 1 to 4, wherein the ionizing radiation element (32) is a Co-60 γ radiation element.

6. The apparatus according to one of claims 1 to 5, wherein the ionizing radiation element (32) provides a radiation dose of 15 Gy

7. A method for growing algae, the method comprising the steps of:
providing a 3-dimensional rack (2);
winding an adherent element (3) on the 3-dimension rack (2);
planting spores (5) of algae in the adherent element (3);
providing a container (1);
filling medium in the container (1);
providing a filter (11) in the container (1) to filter said medium and generate a current of the medium for running-water culturing;
submerging the spores (5) in the medium;
providing a ionizing radiation dose of 15 Gy on the algae for every 3 weeks, wherein the 15 Gy is provided in two rounds; and
providing a running-water pool (6);
submerging the algae in the running-water pool (6) for running-water deuteron-culturing after the spores (5) grow to be sprouts (51); and
picking the algae after the sprouts (51) grow to be properly long bodies.

8. The method according to claim 7, wherein the 3-dimensional rack (2) comprises a plurality of beams and bars (21, 22, 24) connected to one another.

9. The method according to claim 7 or 8, wherein the adherent element (3) is a cotton rope (31).

10. The method according to one of claims 7 to 9, wherein the medium is seawater.

11. The method according to one of claims 7 to 10, wherein the ionizing radiation is a Co-60 γ radiation.

## Patentansprüche

1. Vorrichtung zum Züchten von Algen, umfassend:
einen Behälter (1), der ein Medium zum Züchten von Algen enthält;
einen Filter (121), der in dem Behälter (1) angeordnet ist; wobei der Filter so eingerichtet ist, dass er dazu aktiviert werden kann, das Medium in dem Behälter (1) zu filtern und
eine Strömung des Mediums zu erzeugen;
ein dreidimensionales Gestell (2), das in dem Behälter (1) angeordnet ist;
ein Adhäsionselement (3), das um das dreidimensionale Gestell (2) gewickelt ist, so dass Sporen der Algen in dem Adhäsionselement (3) gepflanzt werden können;
ein ionisierendes Strahlungselement (32), das in dem Behälter (1) angeordnet ist, um die Sporen zu bestrahlen; und
ein Chronometer (33), das an das ionisierende Strahlungselement (32) angeschlossen ist.

2. Vorrichtung nach Anspruch 1, bei der das Medium Meerwasser ist.

3. Vorrichtung nach Anspruch 1 oder 2, bei der das dreidimensionale Gestell (3) eine Vielzahl an Trägern und Stangen (21, 22, 24) umfasst, die miteinander verbunden sind.

4. Vorrichtung nach einem der Ansprüche 1 bis 3, bei der das Adhäsionselement (3) ein Baumwollseil ist.

5. Vorrichtung nach einem der Ansprüche 1 bis 4, bei der das ionisierende Strahlungselement (32) ein Co-60-γ-Strahlungselement ist.

6. Vorrichtung nach einem der Ansprüche 1 bis 5, bei der das ionisierende Strahlungselement (32) eine Strahlungsdosis von 15 Gy bereitstellt.

7. Verfahren zum Züchten von Algen, wobei das Verfahren folgende Schritte umfasst:
Bereitstellen eines dreidimensionalen Gestells (2);
Wickeln eines Adhäsionselement (3) um das dreidimensionale Gestell (2);
Pflanzen von Algensporen (5) in das Adhäsionselement (3);
Bereitstellen eines Behälters (1);
Einfüllen eines Mediums in den Behälter (1);
Bereitstellen eines Filters (11) in dem Behälter, um das Medium zu filtern und eine Strömung des Mediums für eine Kultur in fließendem Gewässer zu erzeugen;
Eintauchen der Sporen (5) in das Medium;
Aufbringen einer ionisierenden Strahlungsdosis von 15 Gy alle drei Wochen auf die Algen, wobei die 15 Gy in zwei Runden bereitgestellt werden; und
Bereitstellen eines Fließwasserbeckens (6);
Eintauchen der Algen in das Fließwasserbecken (6) für eine Fließwasser-Deutero-Kultur nachdem die Sporen (5) zur Sprösslingen (51) herangewachsen sind; und
Ernten der Algen, nachdem die Sprossen (51) zur entsprechenden Länge herangewachsen sind.

8. Verfahren nach Anspruch 7, bei dem das dreidimensionale Gestell (2) eine Vielzahl an Trägern und Stangen (21, 22, 24) umfasst, die miteinander verbunden sind.

9. Verfahren nach Anspruch 7 oder 8, bei dem das Adhäsionselement (3) ein Baumwollseil (31) ist.

10. Verfahren nach einem der Ansprüche 7 bis 9, bei dem das Medium Meerwasser ist.

11. Verfahren nach einem der Ansprüche 7 bis 10, bei dem die ionisierende Strahlung eine Co-60-γ-Strahlung ist.

## Revendications

1. Appareil pour faire pousser des algues comprenant:
un conteneur (1) pour contenir un milieu pour la croissance des algues;
un filtre (121) placé dans le conteneur (1); ledit filtre étant adapté pour être activé pour filtrer ledit milieu dans ledit conteneur (1) et pour générer un courant dudit milieu;
une grille tridimensionnelle (2) placée dans le conteneur (1);
un élément adhérant (3) enroulé sur la grille tridimensionnelle (2) si bien que les spores des algues peuvent être plantées dans l'élément adhérant (3);
un élément de rayonnement ionisant (32) placé dans le conteneur (1) pour irradier les spores; et
un régulateur (33) relié à l'élément de rayonnement ionisant (32).

2. Appareil selon la revendication 1 dans lequel le milieu est de l'eau de mer.

3. Appareil selon la revendication 1 ou 2 dans lequel la grille tridimensionnelle (3) comprend une pluralité de poutrelles et barres (21, 22, 24) reliées les unes aux autres.

4. Appareil selon l'une des revendications 1 à 3 dans lequel l'élément adhérant (3) est une corde en coton.

5. Appareil selon l'une des revendications 1 à 4 dans lequel l'élément de rayonnement ionisant (32) est un élément de rayonnement au Co-60 γ.

6. Appareil selon l'une des revendications 1 à 5 dans lequel l'élément de rayonnement ionisant (32) procure une dose de rayonnement de 15 Gy.

7. Procédé pour faire pousser des algues, le procédé comprenant les étapes de:
prévoir une grille tridimensionnelle (2);
enrouler un élément adhérant (3) sur la grille tridimensionnelle (2);
planter des spores (5) d'algues dans l'élément adhérant (3);
prévoir un conteneur (1);
remplir un milieu dans le conteneur (1);
prévoir un filtre (11) dans le conteneur (1) pour filtrer ledit milieu et générer un courant du milieu pour la culture à l'eau courante;
submerger les spores (5) dans le milieu;
fournir une dose de rayonnement ionisant de 15 Gy sur les algues toutes les 3 semaines,
les 15 Gy étant fournis en deux rations; et
prévoir un bassin d'eau courante (6);
submerger les algues dans le bassin d'eau courante (6) pour la culture à l'eau courante au deutérium après que les spores (5) soient devenus des pousses (51); et
cueillir les algues après que les pousses (51) soient devenues des corps correctement longs.

8. Procédé selon la revendication 7 dans lequel la grille tridimensionnelle (2) comprend une pluralité de poutrelles et de barres (21, 22, 24) reliées les unes aux autres.

9. Procédé selon la revendication 7 ou 8 dans lequel l'élément adhérant (3) est une corde en coton (31).

10. Procédé selon l'une des revendications 7 à 9 dans lequel le milieu est de l'eau de mer.

11. Procédé selon l'une des revendications 7 à 10 dans lequel le rayonnement ionisant est un rayonnement au Co-60 γ.
